# EUROPEAN PATENT SPECIFICATION

(11) **EP 3 355 839 B1**
(45) Date of publication and mention of the grant of the patent: **28.10.2020**
(21) Application number: 16790428.3
(22) Date of filing: 28.09.2016
(51) Int. Cl.: A61F 5/56

(54) **ORTHODONTIC ELASTIC HARMONIZER DEVICE FOR DENTAL-SKULL-FACIAL APPARATUS**
ELASTISCHE KIEFERORTHOPÄDISCHE HARMONISIERUNGSVORRICHTUNG FÜR ZAHN-SCHÄDEL-GESICHTSVORRICHTUNG
DISPOSITIF D'HARMONISATION ÉLASTIQUE ORTHODONTIQUE POUR APPAREIL DENTO-CRÂNIO-FACIAL

(30) Priority: 30.09.2015 IT UB20154030
(43) Date of publication of application: 08.08.2018
(73) Proprietor: Magistro, Francesco, 70010 Capurso (BA) (IT)
(72) Inventor: Magistro, Francesco, 70010 Capurso (BA) (IT)
(74) Representative: Giuliano, Natalia
(86) International application number: PCT/IB2016/055791
(87) International publication number: WO 2017/056010

(56) References cited:
- WO-A2-2012/131208
- DE-A1-102013 108 291
- US-A- 5 462 066
- US-A- 5 876 199
- US-A1- 2009 165 805
- US-A1- 2010 268 107
- US-A1- 2013 029 291

## Description

The present invention relates to an orthodontic elastic harmonizer device for dental-skull-facial apparatus.

In particular, the present invention relates to an orthodontic elastic harmonizer device for dental-skull-facial apparatus, of the type used in dental-skull-facial orthopedics.

It is known that the orthopedic dental-skull-facial orthopedics allows the correct positioning of the facial skeleton, in a perfect neuro-muscle and joint equilibrium (ATM) and aims to eliminate any negative interference that can alter the properly growth determining a dental-skeletal malocclusion. This type of skull-facial orthopedics is addressed to the correction and resolution of the deformations of the bony functional structures of the facial skeleton. The different types of skull-facial unbalance with relative small discrepancies can be determined by problems, most of the times of osteopathic character. In addition, the failure of skull-facial bone structures, in the presence of a malocclusion, must be assessed both to problems related to two grinders (upper dental arch and mandibular arch), and for cranial problems, able to unbalance the ratio between the same arches. For this, as well as the dental arches and skull-facial bone structures, all the muscular structures it must be taken into consideration, and in particular the tongue, the skull muscles and joints. In addition, the masticatory function and mechanisms of growth of the organ of mastication must also be evaluated. In this context, the therapy should be started as early as possible to achieve an adequate "masticatory function" by monitoring during the course of life all the diseases that can alter the patient's masticatory pattern. To achieve this object it is necessary to use orthopedic-functional equipment which modifies the skeletal structure of the craniofacial system and the mandibular posture in a therapeutic sense, improving the function and shape of the skeletal structures that make up the stomatognathic apparatus. In addition, the orthodontic and orthopedic work must be assessed in relation to the shape of the palate and of the skull-facial system.

Moreover, it is to be considered that the position of the mouth and of the head is a component of the postural buoyancy and that each misalignment of the head creates disharmony and tension asymmetries of the neck muscles which are different in relation to the type of deviation. These tension forces are connected to other parts of the underlying trunk muscles and of the overlying facial masticatory muscles, deforming structures and causing malocclusion. In fact, the bones of the skull-facial system can be considered as metamorphosed vertebrae, and there is a structural continuity between the skull and spinal column. This is why a malocclusion can present as a real cranial scoliosis able to determine various dysmetria of the skull-facial thickset, with consequent alteration of the column, thus a postural alteration, having as main cause a disorder of the above structures defined osteopathic injuries.

The relations between the two skull-facial structures and the dental arches are very evident. It is possible also to detect this type of failure by reading the facial aesthetics. When there is a failure it's possible to have a dysmetric facial conditioned by different types of deformation of the skull and face, deviations of the lips, nose, ears, eyes not aligned, and jaws systematically distorted resulting in malocclusions. When there are deviations of this system, the disturbances will be many, which would affect all the skull-facial system and stomatognathic and the whole entire body will suffer the consequences.

The determinant key of the failure according osteopaths, doctors and orthodontists functionalist posturologists, are the deviations of the occiput, a bone positioned posteriorly at the base of the skull, particularly important because to it is connected the spine through the first vertebra of the cervical spine, and the sphenoid, which is also an important bone that is positioned at the center of the skull-facial system, and on which the position of the upper jaw depends, by means of their connection.

In fact, when these two structures, the occiput and the sphenoid, deviate due to a gear incorrectly positioned, defined synchondrosis-spheno-basic (SSB), determine a rotation of the upper jaw, causing various types of malocclusion with related dysmetria of the craniofacial system, through direct connection to the gears of the sphenoid, also diverted to the occiput. The different deviations suffered by occiput and sphenoid are defined lesions, categorized as: bending lesions, extensions lesions, lesions due to torsion, lesion due to lateroflexion-rotation, lesions due to high vertical strain, lesions due to low vertical strain, lateral strain lesions that can occur all from both sides, right and left, and lesion in compression.

Many will be disruption at the level of all the dental-skull-facial structure when there are these lesions, due to deviations of the occiput and of the sphenoid bone of the skull. These hardships can expand to the entire body starting from the spine.

Therefore, the orthodontic and orthopedic devices, adjusting the dental-craniofacial structures, give benefits also to the spine, and thus determine a proper posture.

A known solution is the orthodontic device described in the patent application No. FO2014A000002 assigned to EPTAMED SRL. In particular, the orthodontic device is intended to be interposed between the upper dental arch and the lower dental arch of a user. It is made at least partially with an elastically expandable material, and comprises at least one arc-shaped channel for at least the partial housing of a dental arch. Furthermore, the described orthodontic device comprises at least one position indicator element of a predetermined area designed to be dilated for modify the housing channel.

However, others known dental-facial orthopedic devices, including the one of the above patent application, specificately treat a certain type of skeletal malocclusion related to the underdevelopment of the jaw (second skeletal class with jaw positioned posterior to the upper maxilla), or hyper-development of the jaw (third skeletal class with jaw positioned forward with respect to the upper jaw), or more jaws laterally deviated (shifted to the right or to the left with respect to the upper jaw), and consider only skeletal issues that can be either of vertical origin or sagittal origin (antero posterior movements), or transversal (jaws moved to the right or left with respect to the upper jaw). Therefore, the known devices are not entirely suitable for the solution of skull-facial skeletal problems, namely the deviations of the skeletal structures that make up the skull-facial structure (altering the symmetry), of any entity they are. Furthermore, the device of the Eptamed patent has limits of the type of shape of arch, not taking into account the constitutional aspect of the person and, in addition, has characteristics which are not suitable to the resolution of the different dysmetria of the dental-craniofacial system, because the flanges are not well accolades to the teeth and gingival bone areas, as it should be, are very short and not at the border with the arches. Therefore, it is not able to procure that effect of proprioceptive stimulation of bone matrix, necessary to rebalance dental-skull-facial structures.

In fact, the known devices are not able to mobilize the skull and to restore the architecture of the bony structures of the skull-facial-mandibular system, nor harmonizes the dental arches.

Two known solutions addressing these problems are the patent application DE102013108291 and the patent US5876199. The first one describes an orthodontic appliance comprising: a device body for mounting on the top sheet, wherein the device body an inner wall which is positioned on a lingual side of the top sheet, and an outer wall of the at a cheek side upper arch of the patient is positioned comprises, wherein a web, the inner wall and the outer wall connecting said inner and outer walls each having a top portion protruding beyond the web, to define a receiving channel for the upper dental arch, and the inner and outer walls each having a lower portion which hangs down beneath the ridge, which defines a receiving channel for the lower dental arch; wherein the upper portion of the inner wall of a tongue opening defined to accommodate the tip of the tongue of the patient aware of the upper portion of the inner wall is configured so that when the tip of the tongue of the patient is received, the tip forward the palate is adjacent.

The second one describes an orthodontic appliance for assisting in properly positioning teeth within the mouth of an individual which is capable of fitting various mouth and tooth sizes. The appliance includes a labial-buccal flange, a lingual flange spaced from the labial-buccal flange, both of which define a generally U-shaped configuration in the occlusal view, and an isthmus interconnecting the two flanges. At least one tooth trough is defined between the labial-buccal flange and the lingual flange for receiving either an upper or a lower row of the individual's teeth. The appliance includes no individual tooth sockets but instead utilizes pressure applied by the labial-buccal flange, the lingual flange, and the relative angles and material thicknesses to properly position the teeth. The appliance is capable of fitting mouths and teeth of various sizes because it includes no individual tooth sockets. The appliance is also useful as a device for preventing sleep apnea, snoring and bruxism as well as an athletic mouth guard.

A problem of these known solutions is that do not perform a correct re-education of the tongue and swallowing, when these problems are present. In fact, the tongue has a role of primary importance in the mouth, because it is able to influence the morphology of the dental arches and above all is able to create disharmony. People who breathe through the nose have the tongue positioned down instead of being on the palate and every swallowing expands the palate and contributes to respiratory primary craniosacral motion (CSP). Instead, who breathe through the mouth have the tongue placed at the bottom instead of the top against the palate. In this position the tongue is not able to stimulate the growth of the same and also being interposed on the teeth causes various types of malocclusion and damage to the periodontium sometimes even irreversible for the loss of the front teeth. However, when the tongue is in the correct posture and the swallowing is correct, that stabilizes the skull-column ratio, conforms the alveoli-dental relationships, balancing relations between the sphenoid and occiput. The tongue not compacting the palate and determining an incorrect swallowing can interfere with all of these receptor systems, providing postural changes.

The object of the present invention is to provide an orthodontic elastic harmonizer device for dental-skull-facial apparatus able to ensure a respectful adaptation occlusal scheme, in relation to skull-facial pattern, performing a "purely orthopedic" treatment which harmonizes the skull-facial skeletal structures, for the restoration of several dysmetria and deflections of the skull and face and able to allow a re-education of the poor swallowing, able to overcome the limitations presented by the known devices.

According to the present invention, an orthodontic elastic harmonizer device for dental-skull-facial apparatus is provided, as defined in claim 1.

For a better understanding of the present invention it is now described preferred embodiments of the disclosure which do not necessary comprise all the features of the invention, purely by way of non-limiting example, with reference to the accompanying drawings, in which:
- Figures 1A-1B show respectively a front view and a side view of a first embodiment of an orthodontic elastic harmonizer device for dental-skull-facial apparatus;
- Figure 2 shows a rear view of the first embodiment of the orthodontic elastic harmonizer device for dental-skull-facial apparatus;
- Figures 3A-3B show views respectively of the inner walls of the upper arch and of the lower arch of the first embodiment of the orthodontic elastic harmonizer device for dental-skull-facial apparatus;
- Figure 3C shows an occlusal plane of the first embodiment of the orthodontic elastic harmonizer device for dental-skull-facial apparatus;
- Figures 4A-4B shows respectively a front view and a side view of a first alternative of the first embodiment of the orthodontic elastic harmonizer device for dental-skull-facial apparatus;
- Figure 5 shows a rear view of the first alternative of the first embodiment of the orthodontic elastic harmonizer device for dental-skull-facial apparatus;
- Figures 6A-6B show views respectively of the inner walls of the upper arch and lower arch of the first alternative of the first embodiment of the orthodontic elastic harmonizer device for dental-skull-facial apparatus;
- Figures 7A-7B shows respectively a front view and a side view of a second alternative of the first embodiment of the orthodontic elastic harmonizer device for dental-skull-facial apparatus;
- Figure 8 shows a rear view of the second alternative of the first embodiment of the orthodontic elastic harmonizer device for dental-skull-facial apparatus;
- Figures 9A-9B show views respectively of the inner walls of the upper arch and lower arch of the second alternative of the first embodiment of the orthodontic elastic harmonizer device for dental-skull-facial apparatus;
- Figures 10A-10B show respectively a front view and a side view of a third alternative of the first embodiment of the orthodontic elastic harmonizer device for dental-skull-facial apparatus;
- Figure 11 shows a rear view of the third alternative of the first embodiment of the orthodontic elastic harmonizer device for dental-skull-facial apparatus;
- Figures 12A-12B show views respectively of the inner walls of the upper arch and lower arch of the third alternative of the first embodiment of the orthodontic elastic harmonizer device for dental-skull-facial apparatus;
- Figures 13A-13B show respectively a front view and a side view of a second embodiment of the orthodontic elastic harmonizer device for dental-skull-facial apparatus;
- Figure 14 shows a rear view of the second embodiment of the orthodontic elastic harmonizer device for dental-skull-facial apparatus;
- Figures 15 show views respectively of the inner walls of the upper arch and lower arch of the second embodiment of the orthodontic elastic harmonizer device for dental-skull-facial apparatus;
- Figure 16 shows respectively a front view and a side view of a third embodiment of the orthodontic elastic harmonizer device for dental-skull-facial apparatus;
- Figure 17 shows a rear view of the third embodiment of the orthodontic elastic harmonizer device for dental-skull-facial apparatus;
- Figures 18A-18B show views respectively of the inner walls of the upper arch and lower arch of the third embodiment of the orthodontic elastic harmonizer device for dental-skull-facial apparatus;
- Figure 19 shows an occlusal plane of the second embodiment of the orthodontic elastic harmonizer device for dental-skull-facial apparatus;
- Figure 20 shows an occlusal plane of the third embodiment of the orthodontic elastic harmonizer device for dental-skull-facial apparatus;
- Figures 21A-21B show respectively a side view and a rear view of a fourth embodiment of the orthodontic elastic harmonizer device for dental-skull-facial apparatus;
- Figure 22 shows a view of the inner walls of the top arch of the fourth embodiment of the orthodontic elastic harmonizer device for dental-skull-facial apparatus;
- Figures 23A-23B show respectively a side view and a rear view of a first alternative of the fourth embodiment of the orthodontic elastic harmonizer device for dental-skull-facial apparatus;
- Figures 24A-24C show respectively a perspective view and views of the inner walls of the upper arch and lower arch of the first alternative of the fourth embodiment of the orthodontic elastic harmonizer device for dental-skull-facial apparatus;
- Figure 25 shows an occlusal plane of the first alternative of the fourth embodiment of the orthodontic elastic harmonizer device for dental-skull-facial apparatus;
- Figures 26A-26C show occlusal guides of the first, second and third embodiment of the orthodontic elastic harmonizer device for dental-skull-facial apparatus.

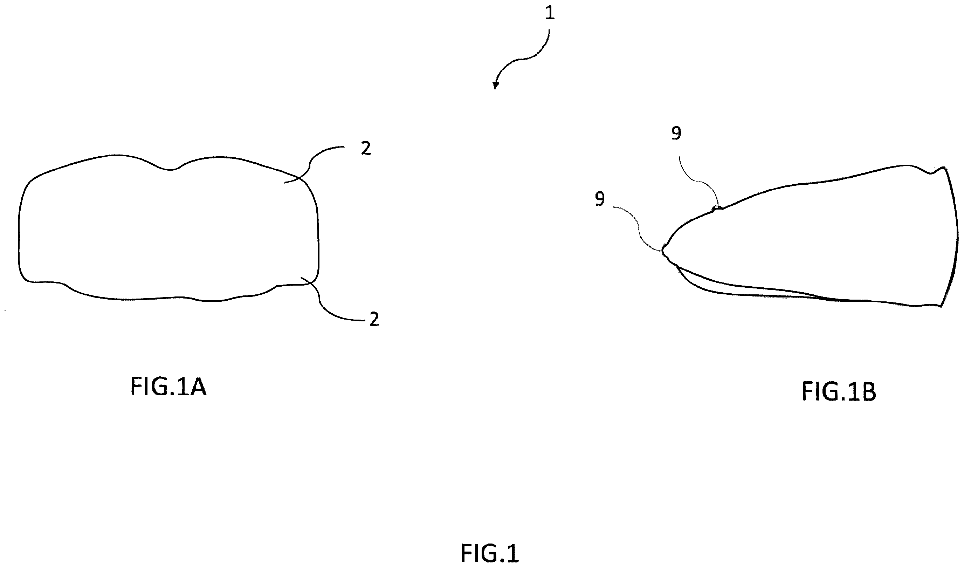

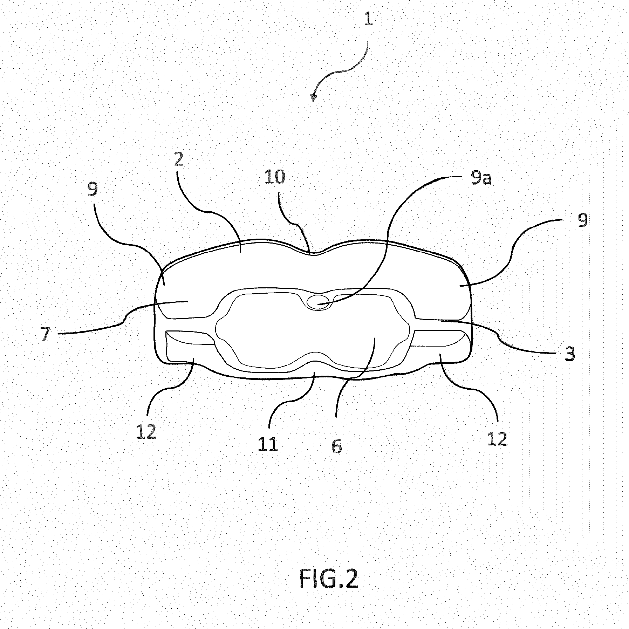

With reference to these figures and, in particular, and to figure 1-3, a first embodiment of an orthodontic elastic harmonizer device for dental-skull-facial apparatus is shown, according to the disclosure. More in details, the orthodontic elastic harmonizer device for dental-skull-facial apparatus 1 consists essentially of an occlusal bimaxillary (maxillary and mandibular) apparatus which is elastically deformable, provided with a pair of top and bottom guides 2 for the dental arches. In particular, the pair of guides 2 comprises external guides 2, defined vestibular flanges which cover the upper and lower teeth, as well as of the entire vestibular gingival surface extended to the area of the fornices, and internal guides 6 defined lingual-palatal flanges and carry out a full coverage at a gingival level, mandibular level, tongue level and partial palate level. As shown in figure 2, the two arches are connected by an occlusal plane 3, which is also elastically deformable, which may be subject to a chewing operation by the person who uses it, allowing, therefore, to rub and gently massage the areas in contact with the flanges 2. In this way, it produces a series of stimuli directed to obtain muscle-relaxant action of the neuro-muscular system (muscles and innervation), and to the gingival and bone reconditioning (periodontal).

On the back of the upper surface of the occlusal plane 3 of the device 1, shown in Figure 2, a first and a second groove are provided laterally, the first groove in the corners between the occlusal plane 3 and the vestibular flanges 2 and the second groove between the occlusal plane 3 and the lingual flanges 6, in order to position, interlocking on the upper surface of the occlusal plane 3, insertable and removable bands, having a thickness for example equal to approximately 2 mm, to make changes of the occlusal planes of the device 1.

Furthermore, on the edges of the back area of the upper part of the flanges 6, corresponding to molars or sixths, there are provided two protruded buttons 9 as a measurement reference of the device 1, useful to detect the size of the device in relation to the extent of the arch of the patient.

The device 1 also includes the inlets, better defined as elements 10a, 11, 12, to unload the labial, lingual and laterals frenulum; these allow the correct positioning respectively with the labial frenulum, lingual frenulum and with that side ones.

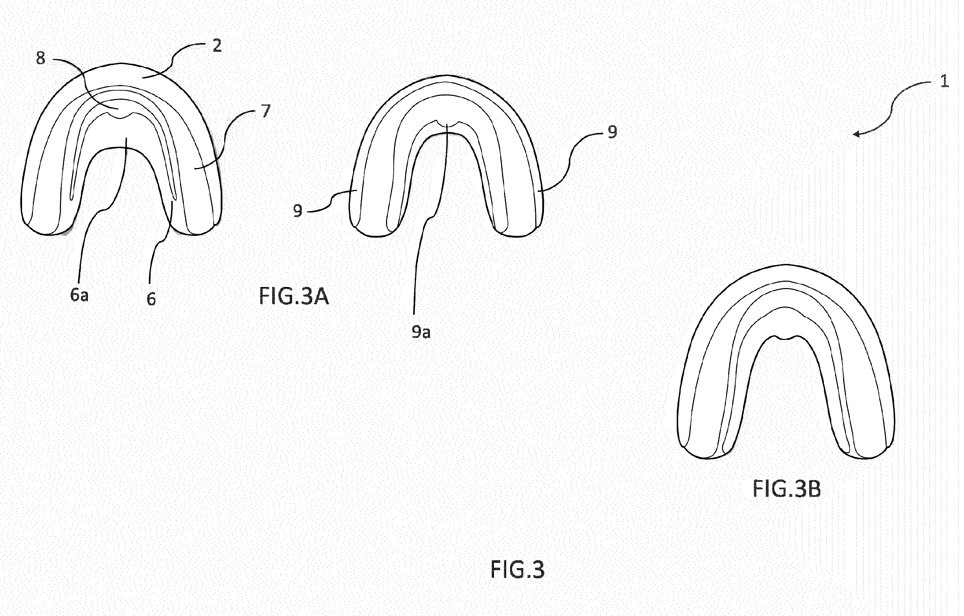

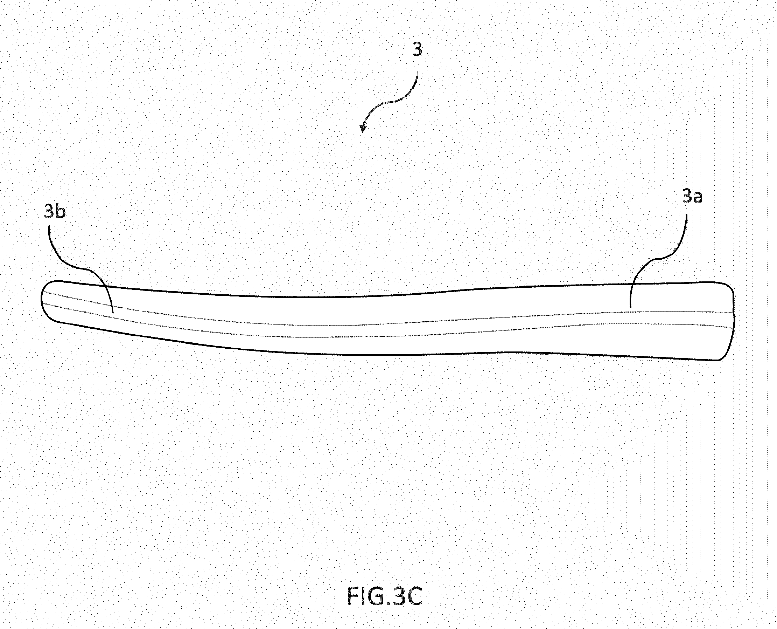

As shown in Figure 3, the internal part of the device 1, ie the one in contact with the tongue, is also molded with a pair of lingual-palatal internal flanges 6 (palatal in the top part and tongue in the bottom part) having height and footprint smaller than the flanges 2 to cover the upper and lower arches described previously.

Again with reference to figure 3, it can be noted that the palatal upper flange 6 is provided with a concave area 8 used to unload the back incisive papilla. In fact, the postural receptors are located in this area of the palate and need to be always stimulated by the tongue of the patient using the device 1 and the concave area 8 leaves entirely free the area of the palatine postural receptors for their stimulation under the action of the device 1, not compressing the back incisive papilla and avoiding any grazes.

Moreover, the upper palatal flange 6 comprises a steep inclined plane 6a for the positioning of the tongue and a button 9a for guiding the tongue, when there is an atypical swallowing problem with low posture, or diverted, rotated, thus allowing reeducation exercises of the tongue for proper swallowing. Also the inclined plane 6a, which extends from the plantar back incisive palatal upper edge to the border of the lingual frenulum, is configured to provide a support to the tongue for guiding it toward the little button 9a in addition to indicated to act as a shield to the interposition of it on the teeth. Thus the tongue is exerted to a correct posture (spot) during the rest phase and to a correct swallowing, through a peristaltic movement towards the palate.

This type of steep inclined plane that extends up to the frontal lower teeth is designed with a triple function, as it also acts as a shield for the interposition of the tongue on the teeth, also determining the repositioning of the badly positioned teeth (lower incisors) when these are arrears, through a gentle push, with the purpose to guide these in a correct alignment position.

In the concave volume 7 bounded by the two external vestibular flanges 2, by the palatal-lingual internal 6 and by the occlusal plane 3 that serves as a connection means, the patient's teeth using the device will be placed.

Advantageously according to the disclosure, the device 1 is particularly designed for a rounded face shape nearest to a circular shape.

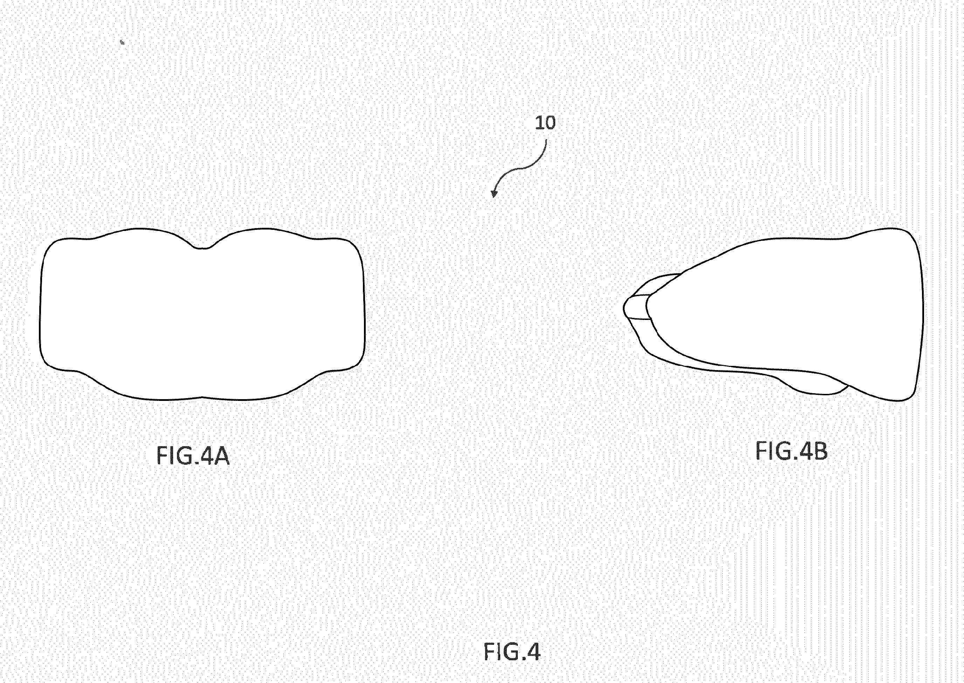

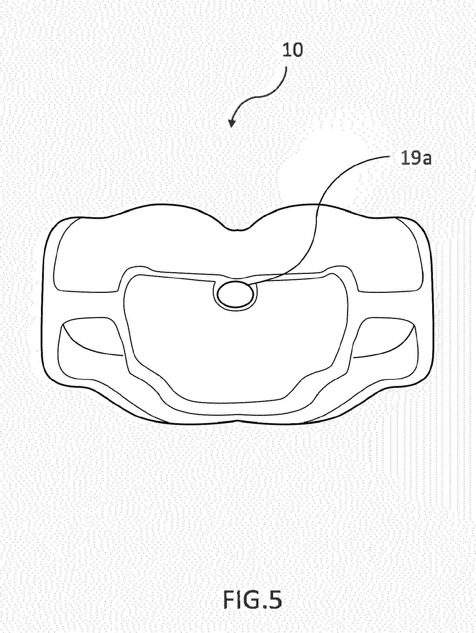

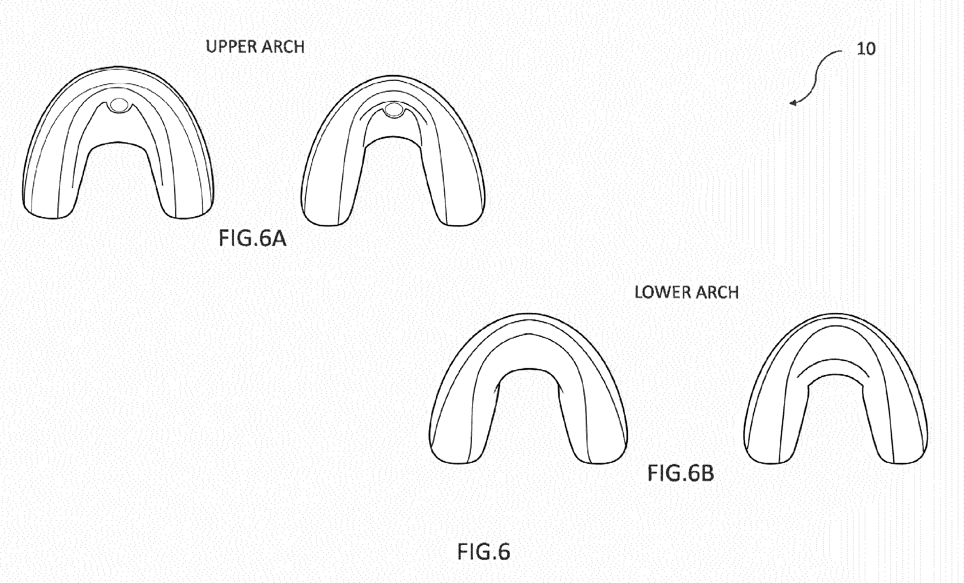

Figures 4-6 show a device 10 as a first alternative of the device 1 and is particularly designed for a rounded face shape nearest to an ogival shape.

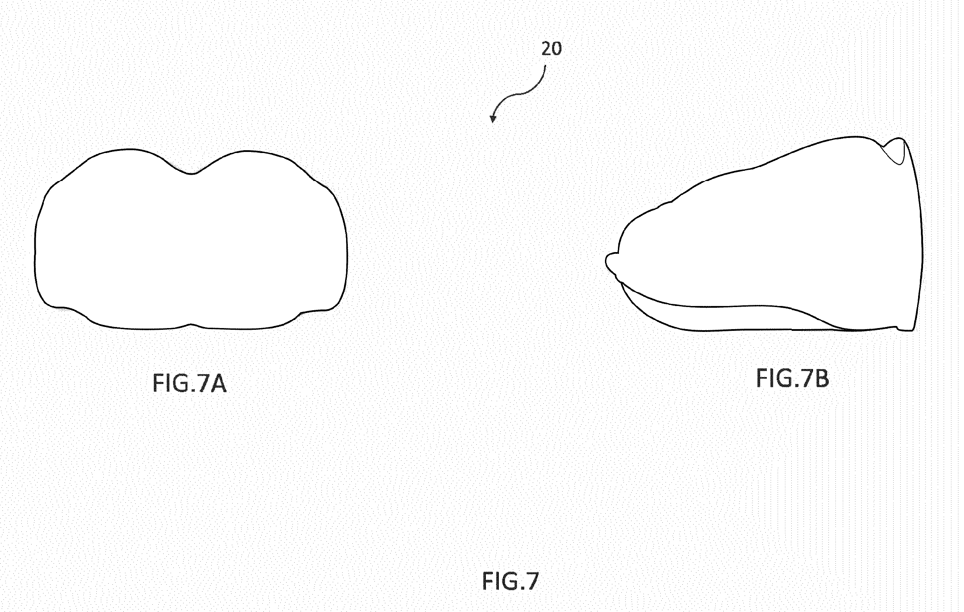

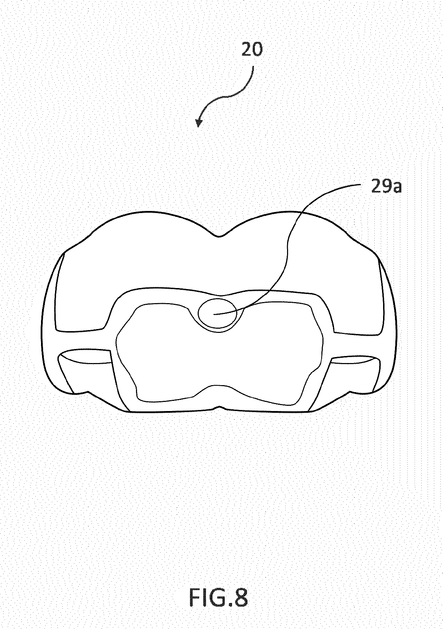

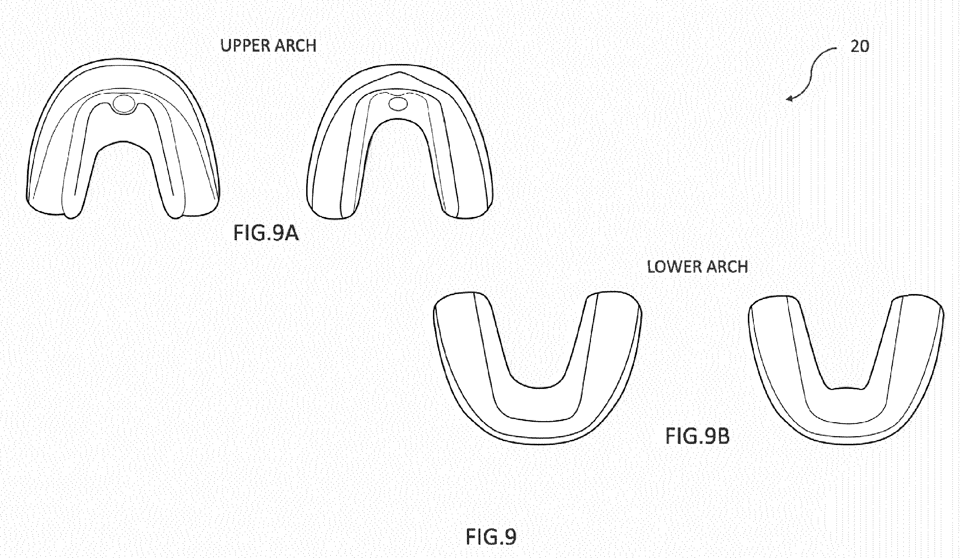

Figures 7-9 show a device 20 which is a second alternative of the device 1 designed particularly for a face shape nearest to a rectangular shape.

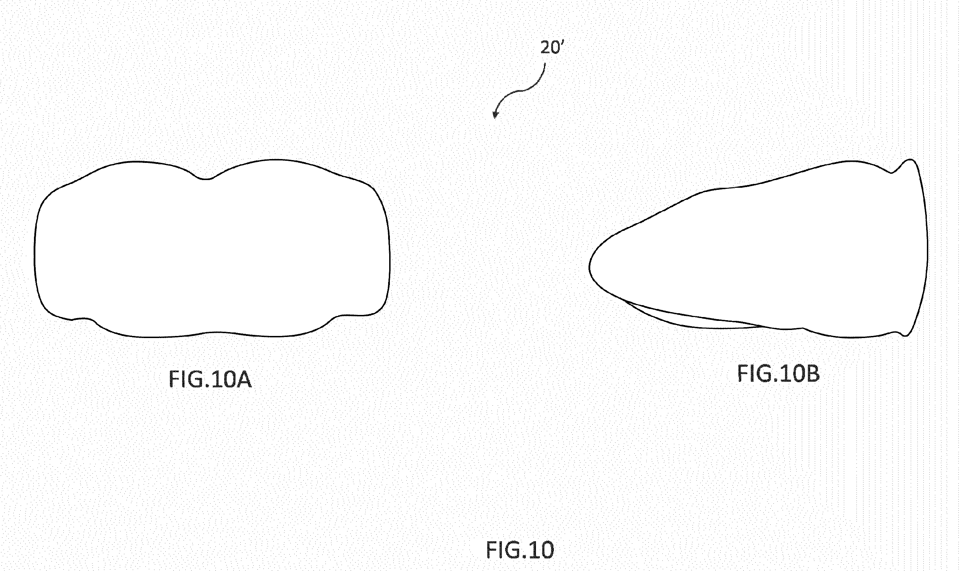

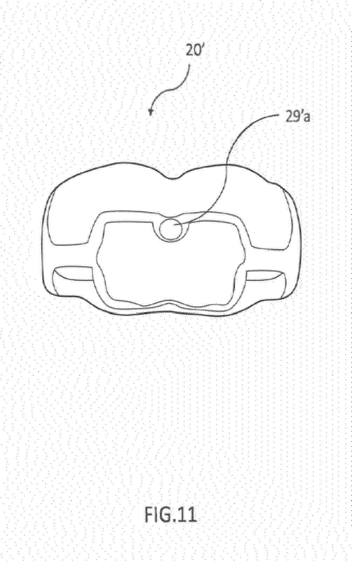

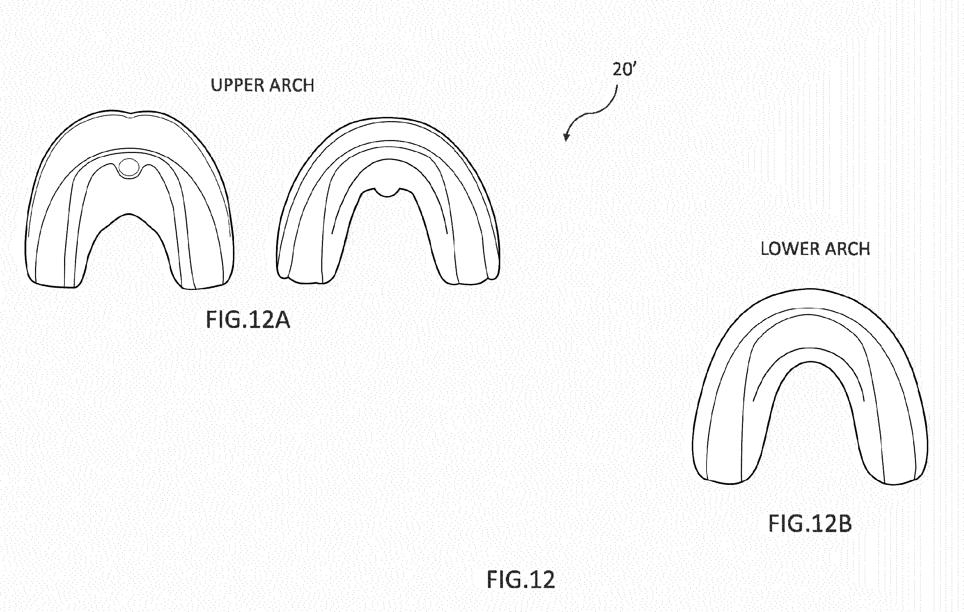

Figures 10-12 show a device 20' which is a third alternative of the device 1 particularly designed for a normally rounded shape of the face that reflects the constitution of a normal person.

All the devices 1, 10, and 20 and 20' are considered usable for the first skeletal class, or for situations of correct position of the occlusal relationships of the upper arch with the antagonist lower arch. In particular, the devices 1, 10, and 20 and 20' comprise the vestibular flanges 2 and the lingual flanges 6 well accolades to the gingival ridges and well extended almost to the border with the arches.

According to an aspect of the disclosure, the vestibular upper flanges 2 have a thickness comprises between 1,5 mm, in the border area near the arches for a proprioceptive stimulation of bone matrix, and 3 mm descending towards the central area of the occlusal plane 3, and while the vestibular bottom flanges 2 have a thickness just major than the vestibular upper flanges 2 and extend, in correspondence of the lower front teeth, over the teeth of about 5/7 mm, so as to provide a lip-shield effect for reconditioning muscle of the lower lip. This arrangement is particularly useful when there isn't a good muscle lip tone, due to loss of the vertical dimension of the mouth.

According to another aspect of the disclosure, the rear bottom vestibular flanges 2 are well attached and shaped to the teeth and extend approximately 2.4 mm beyond the teeth and extend in length to the border with the last rear positioned molars. The upper palatal flanges, however, are on the border with the palate with minimum back incisive palatal support, defined plantar area, with a thickness that varies from 3 to 5 mm, with consequent expansive effect in the upper arch.

According to another aspect of the disclosure, the bottom tongue flanges 6 are well attached to the teeth and extend beyond, in contact with the gingival bone area of about 3-4 mm and have a thickness comprised between 3 and 5 mm to provide a good expansive boost of lower arch.

From the lingual shield having a steeply inclined plane 6a the two side lingual flanges 6 extend, well accolades to the teeth, and stretch until the last rear positioned molars. The two vestibular flanges 2 and lingual flanges 6 are accolades between them from the occlusal plane 3, so as to form a guide relating to a type of arch shape such as to form a guide track to the teeth toward the external and internal walls of gingival ridges. The thickness of the guides varies from about 2 mm, in the area of the upper front teeth (incisors canines), up to 5/6 mm in the area of the posterior teeth (premolars), and up to 10 mm in the rear area of the molar teeth. Instead, the guides of the lower arches are the same, but approximately 1 mm narrower. This type of track is crucial to the proper repositioning both of the skeletal bases of the dental arches and of teeth, by means of the attachment and stimulation (proprioception) of the flanges well-necked to the teeth and also at bone level (periodontal).

Advantageously according to the disclosure, the type of the occlusal plane that connects the two flanges is designed with characteristics relating to the individual's natural physiology, as they represent the support of the joints (ATM) to obtain a proprioceptive contact, relative to the curves and to rises that characterize them. In fact, the occlusal plane of the first class orthodontic devices 1, 10, 20, 20' has, as shown in Figure 3C, a front occlusal portion 3a more raised, for example of about 2 mm, in the front area with respect to the back occlusal portion 3b. This ensures, together with an elastic effect procured by the type of elastic material used, the extrusion stimulation of the posterior teeth, with relative upward of the vertical dimension of the mouth, as it is one of the crucial components in the success of the treatment, freeing the jaw and then the ATM, with the consequent improvement of both skeletal and muscle problems. Moreover, for the majority of skeletal malocclusions that are determined by deep bites, or by the reduction of the vertical dimension of the mouth, the posterior occlusal planes have a shape with adequate curves, in relation to the Spee and Wilson functional curves for a proper jaw growth.

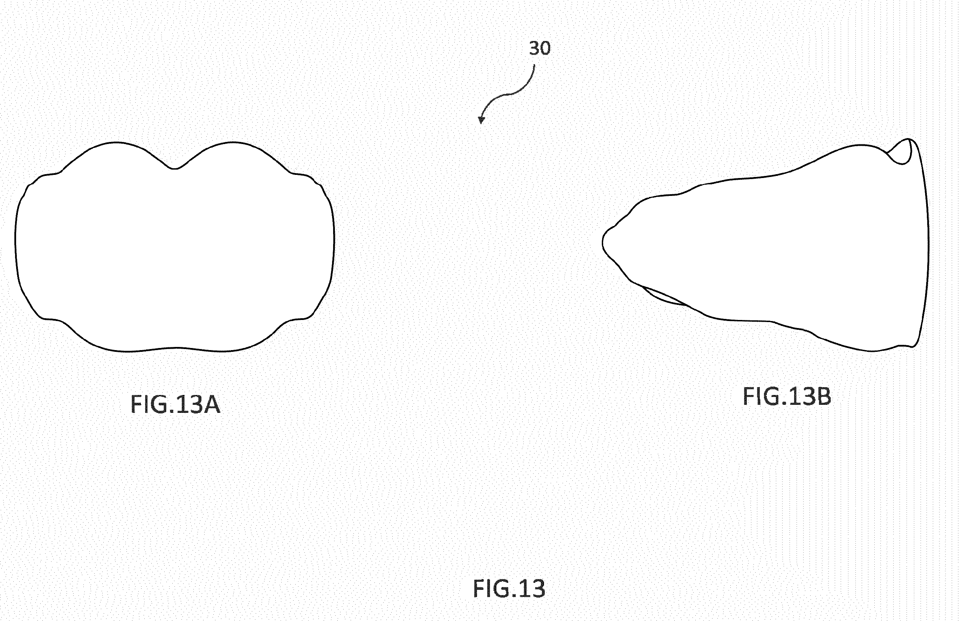

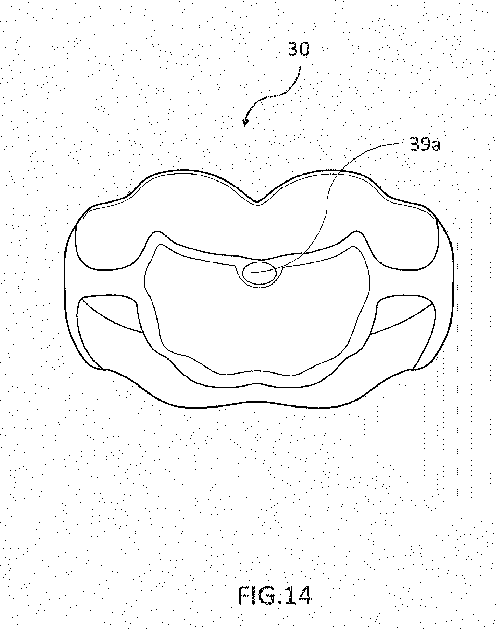

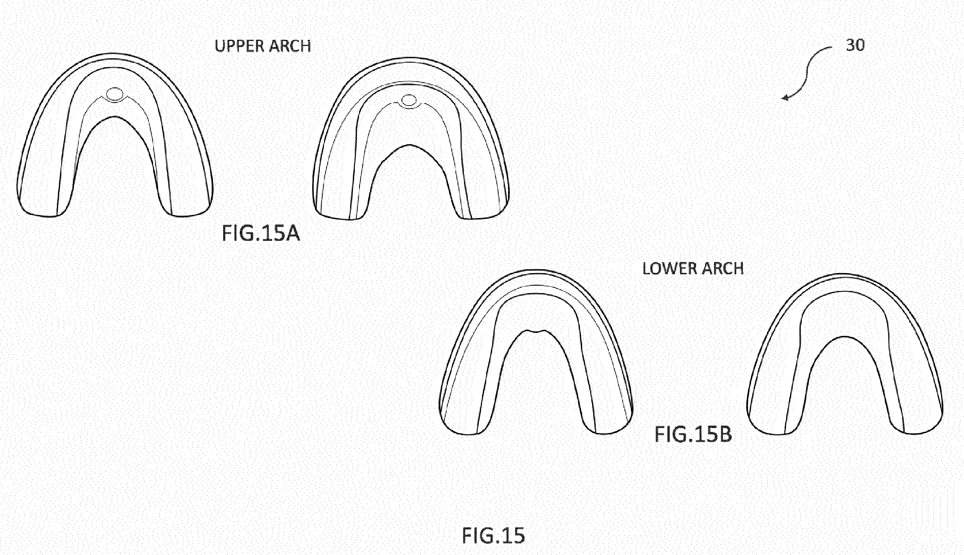

Figures 13-15 show a second embodiment of the orthodontic elastic harmonizer device for dental-skull-facial apparatus, according to the disclosure. The device 30, shown in Figures 13-15, is particularly designed to solve the problem of malocclusion referred to the second skeletal class, which shows a very pronounced deep bite associated with a retruded mandible, or mandible positioned behind with respect to the normal one, with lower incisors that are positioned back and with the edges incisors against the palate, while the upper incisors are very advanced and fanned forward. This skeletal conformation is such as to provide a big incompetence of the lips, compromising the neuro-muscular system (muscle tension due to the muscle tone loss and the masticatory muscle contracture), and joint (ATM) (failure of the joints due to a jaw blocked by the deep bite) with negative consequences and a very uncomfortable facial aesthetics. Moreover, in such cases, the tongue is positioned at the bottom instead of against the palate for lack of space in the oral area, with abnormal swallowing compromising the mastication and phonation.

The orthopedic device 30 for second skeletal classes have the same characteristics of the devices 1, 10, 20, 20' but it is designed, in particular, for the recovery of the correct position of the jaw, through the advancement of the same (by means of a well-defined occlusal plane guide), with the purpose of positioning the dental arches (upper maxilla and lower mandible) in a correct first class skeletal location, taking into account the addition of other components which improve its function, in order to obtain benefits both at the functional (muscle reconditioning and articular) and aesthetically level.

Advantageously according to the disclosure, the occlusal plane of the device 30 for the second skeletal classes is slightly raised in the front area (incisal-canine area) with respect to the occlusal plane of the first class. In this way, the vertical dimension is further raised, for relaxing the masticatory muscles and improve, therefore, the neuro-muscular system. Through the steep inclined plane 6a the jaw is conditioned in protrusive position (forward position). Then, to stabilize it in this configuration, specific stimulation exercises should be performed by the patient during the day and the device must also be held during the night.

Advantageously according to the disclosure, the orthodontic device 30 has very high vestibular flanges 2 on the border with the upper arches, and, in the area of the upper front teeth, have an indent effect, namely more accolade to the fornix, because of the flaring of the upper front teeth (incisors) who generally have the tendency to remove the flange from the contact with the fornix, at the same time interfering with the upper lip.

In addition, the orthodontic device 30 provides the lower vestibular flange 2 which is also well-necked to the teeth along its entire circumference, but, in the area of the lower teeth (incisors), much more extended almost to the border with the lower fornix (for example, acting as a bottom lips shield), to remodel and make more tonic the usually very hypotonic lower lip also through specific exercises. The lingual flange 6 is also well-necked to the teeth and also extends over, especially in the central rear tongue area with the tongue shield and the steep inclined plane to guide the tongue in a correct posture towards the spot and with the help of the little button to act as a stimulus, through specific exercises (speech therapy), considering that the tongue participates, and sometimes it is also crucial (when it is in a correct position and with regular swallowing) for the reconditioning of the joints (ATM). In the central area of the insole at the border with the little button there is the small concavity do not interfere with the back incisive papilla, while the two vestibular and lingual flanges connect to the occlusal plane and are well bonded to the teeth and the gingival ridges for an orthopedic effect for the symmetry of the dental arches and of the device, determining at the same time a good competence of the lips and the recovery of a neuro-muscular system (usually uncontrolled), and for a function and a better aesthetic.

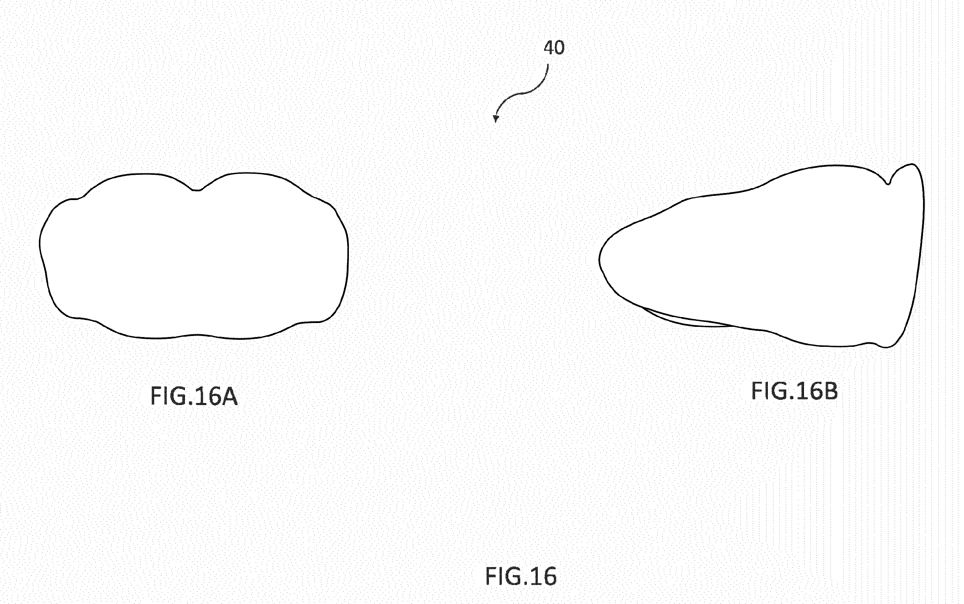

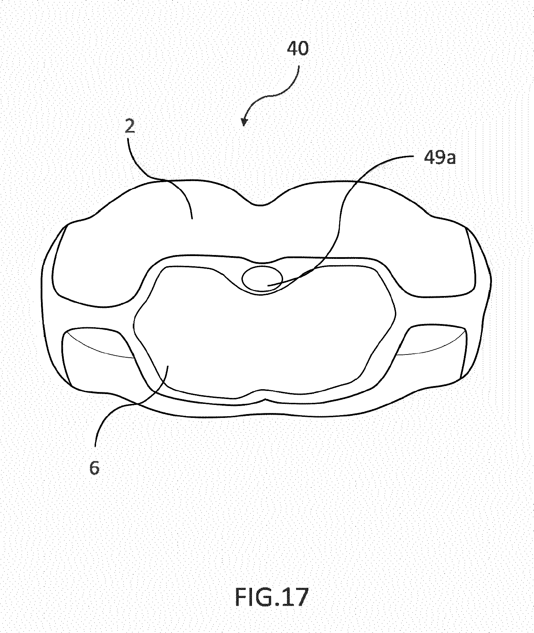

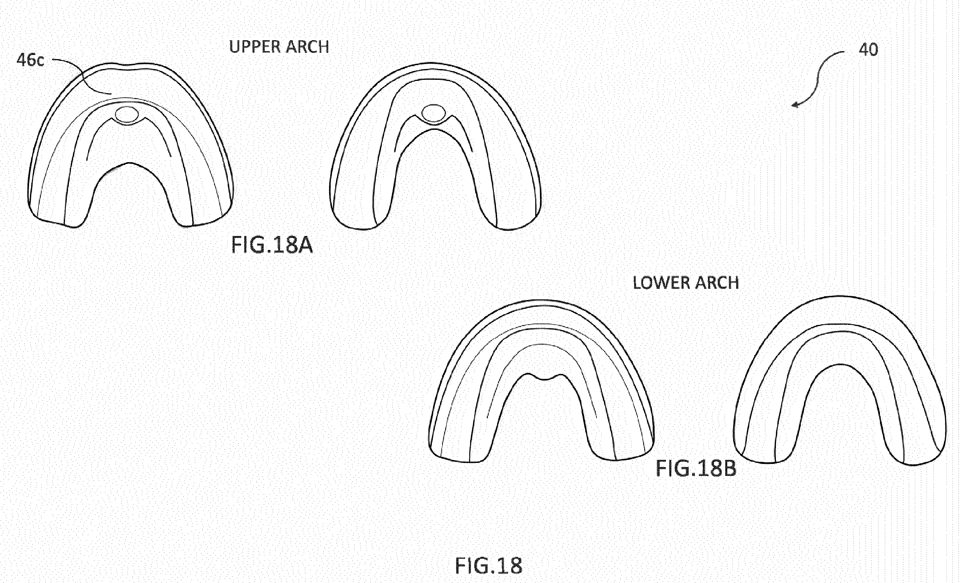

Figures 16-18 show a third embodiment of the orthodontic elastic harmonizer device for dental-skull-facial apparatus, particularly intended for the third skeletal classes. In particular, the device 40 is particularly designed for the third skeletal classes. This type of skeletal class is characterized by a malocclusion originated by a functional nature, articular (ATM) and of the cervical spine, and aesthetically involves a great discomfort for the patient. The mandible is in protruded position, namely placed forward compared to the normal one, with the upper incisors in the opposite position with respect to the lower incisors (crossbite), resulting in an abnormal occlusal relationship and, therefore, a severe malocclusion because of wrong movement in sagittal (anterior-posterior) of the two jaws. One of the main causes of malocclusion of skeletal nature of the third functional classes is the atypical swallowing, with low posture of the tongue thrusting on the lower teeth and a force of abnormal thrust exerted at a mandibular level, which causes the growth of the same in the protruded position, as well as the flaring of the teeth, especially the lower incisors, sometimes causing serious damage at bone periodontal level with mobility of the same. The orthodontic elastic harmonizer device for dental-skull-facial apparatus 40 for the third-skeletal classes is designed for the treatment of mandibular pseudo-prognazie positioning the dental arches in a regular incisors ratio, by means of design of a very specific occlusal plane to the type of skeletal class. In particular, the upper teeth are positioned on a posterior-anterior sliding plane and, on the contrary, by exerting pressure for a braking action of the jaw through a labial shield 46c, shown in Figure 18, of the lower front flange 6, which is very necked to the teeth and extends downward to the lower fornix, with the purpose to determine a pair of inverse force for a combined effect, in addition to the recovery of a correct position of the upper jaw and of the lower jaw in the first class, also the symmetry of deviations of the device, when these are present. More specifically, the orthodontic elastic harmonizer device for dental-skull-facial apparatus 40 for third class presents vestibular flanges 2 and lingual-palatal flanges 6 well accolades to the teeth for a harmonizing effect of the dental arches. In addition, the vestibular flange 2 is well oriented, high, on the border with arches but in the front area of the front teeth (premaxilla). The flange 2 is slightly detached from the gingival bone area, such as to form a sort of upper lips shield. This feature responds to the possible presence of a bone retraction in the same area, due to a very hypertonic lip which does not allow the development. Eliminating the lip pressure is possible the bone growth and development in the area of the premaxilla and simultaneously stimulate the lip to a regular tone. Instead, the lower vestibular flange 2, in the area of the front anterior teeth, is well accolade to the teeth, as to form a containment shield of the mandibular growth. Furthermore, the vestibular flange 2 is well accolade to the teeth and also extends over, especially in the central rear tongue area, with the steep inclined plane to guide the tongue in a correct posture toward the spot and with the help of the little button to act as a stimulus through specific exercises (speech therapy), eliminating one of the underlying causes of the teeth unbalance. In the front central area of the insole, at the border with the little button the small concavity is present do not interfere with the back incisive papilla.

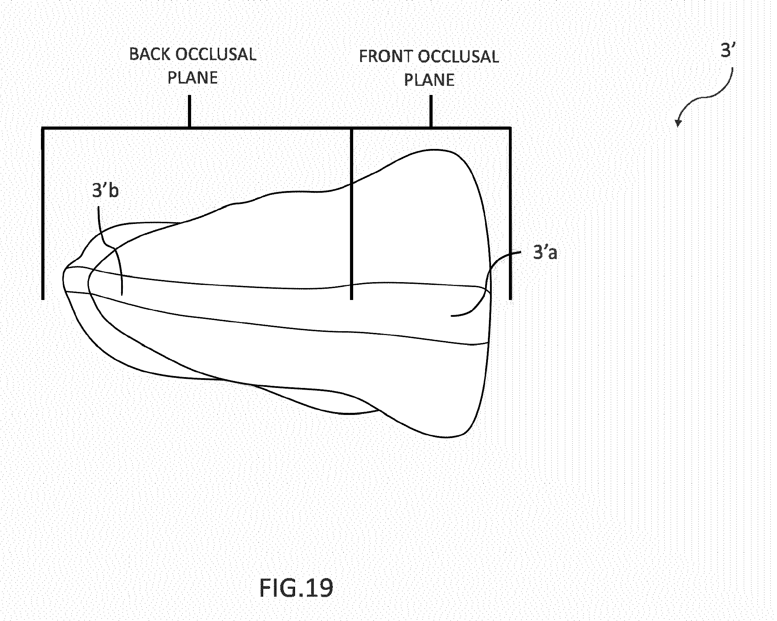

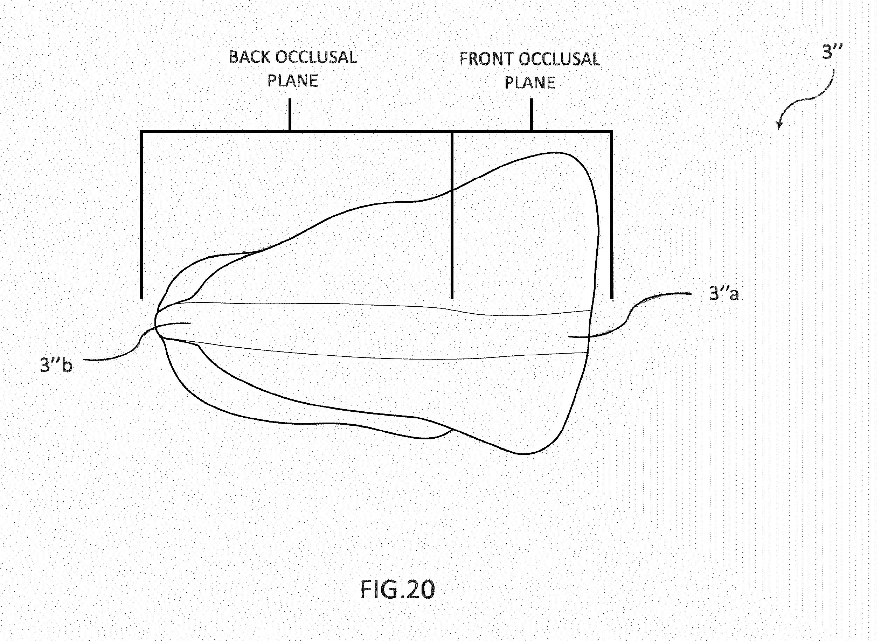

Figure 19 shows the occlusal plane 3' for the second skeleton classes in which the front occlusal plane 3'a is slightly higher with respect to the back occlusal plane 3'b. The occlusal plane 3" for third-skeletal classes, shown in figure 20, in contrast to the occlusal plane for the first class and second class, has a plan differently designed, because the back occlusal plane 3"b is slightly higher than the occlusal front plane 3"a (incisive canine area) with sliding effect toward the front of the upper jaw compared to the mandibular occlusal. Finally, the two vestibular flanges 2 and lingual flanges 6 are connected to the occlusal plane resulting adherent to the teeth for an orthopedic effect, placing the dental arches in the first skeletal class and for a correct tongue posture and swallowing as well as for an orthopedic symmetric effect of the dental arches and, therefore, of the craniofacial system, ensuring a good competence of the lips for a better function and appearance.

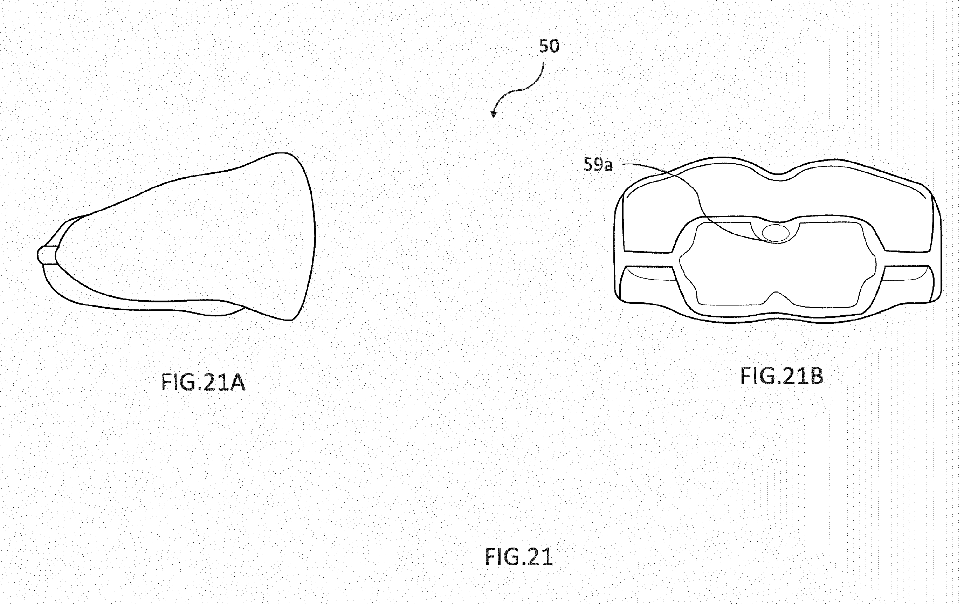

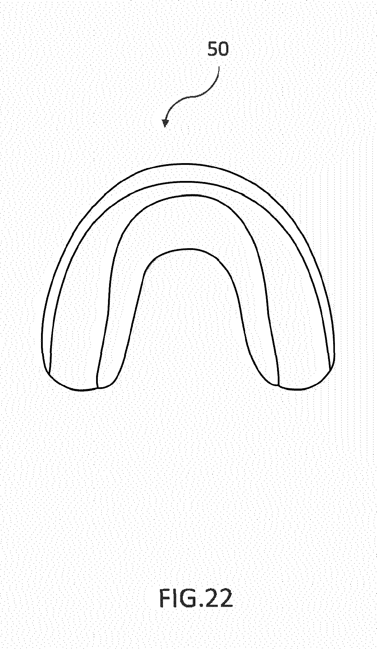

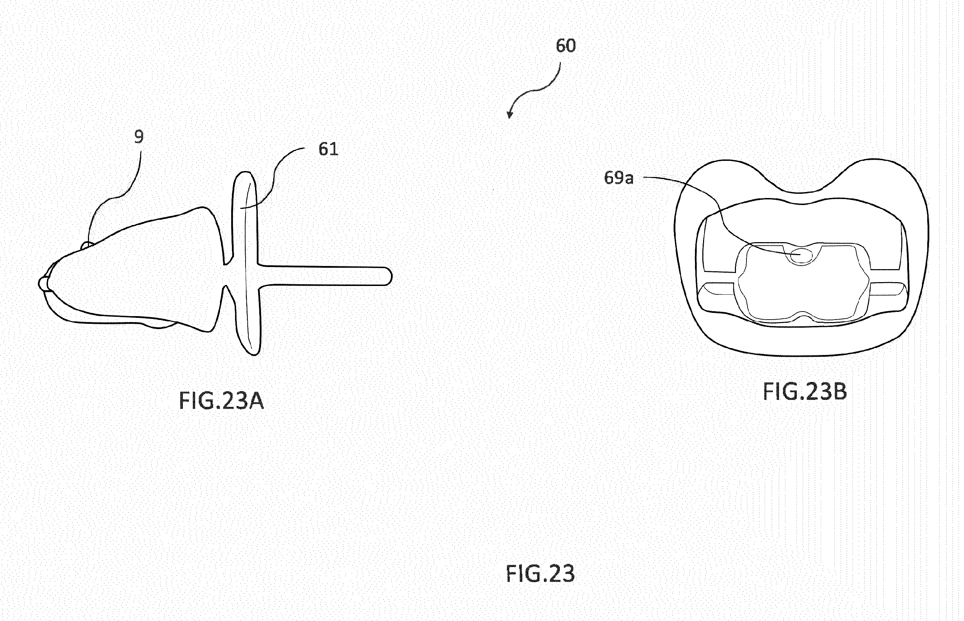

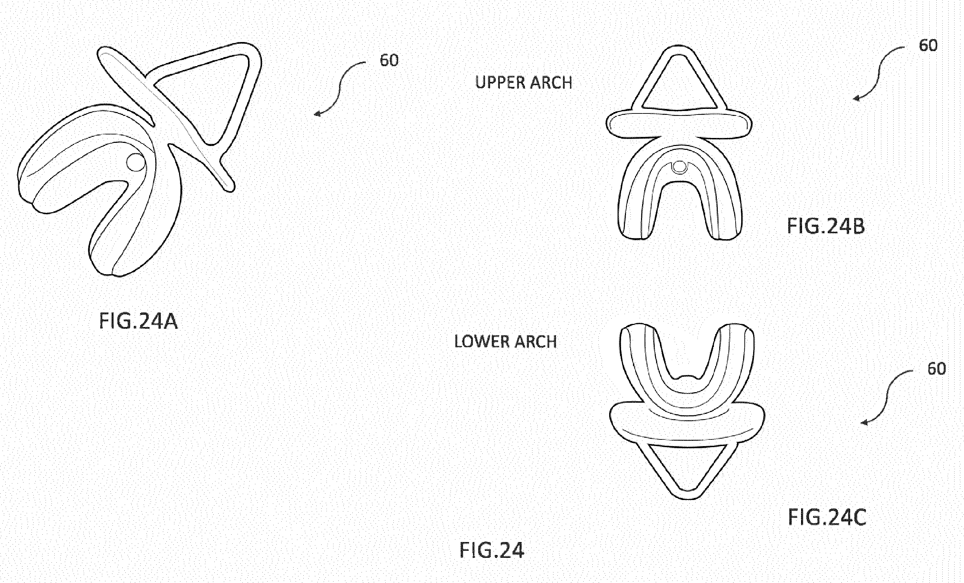

The figures 21-22 show a fourth embodiment of the orthodontic elastic harmonizer device for dental-skull-facial apparatus and Figures 23-24 show a first alternative of the fourth embodiment of the orthodontic elastic harmonizer device for dental-skull-facial apparatus, according to the disclosure. Both devices 50 and 60, shown respectively in Figures 21-22 and 23-24, are particularly designed for very young children with completely deciduous teeth (milk teeth), with the same characteristics of the first class orthopedic devices, when there are deviations and defects of the dental arches, associated with deviations of the craniofacial system. These devices provide vestibular flanges 2 and lingual flanges 6 well necked on the teeth and on the gingival ridges, and the tongue shield with the steep inclined plane is always present to direct the tongue to the spot. There is also the housing for the back incisive papilla in the central area of the insole.

Moreover, advantageously according to the disclosure, the devices 1, 10, 20, 20', 30, 40, 50, 60 have a tab 9a, 19a, 29a, 29'a, 39a, 49a, 59a and 69a to further stimulate the tongue toward the spot to encourage kids to a correct swallowing.

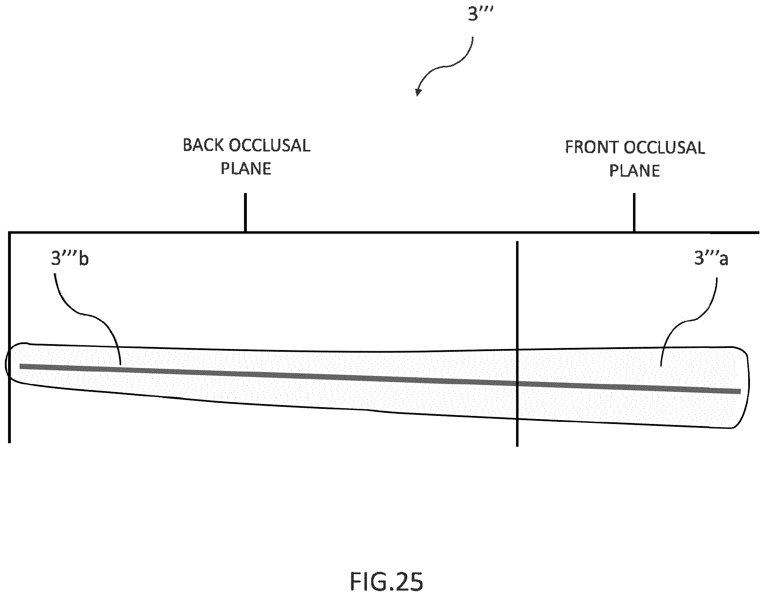

Advantageously according to the disclosure, as shown in Figure 25, the occlusal plane 3''' of the devices 50 and 60 is perfectly horizontal without Spee and Wilson curves, in consideration of the non-presence of the first permanent molar.

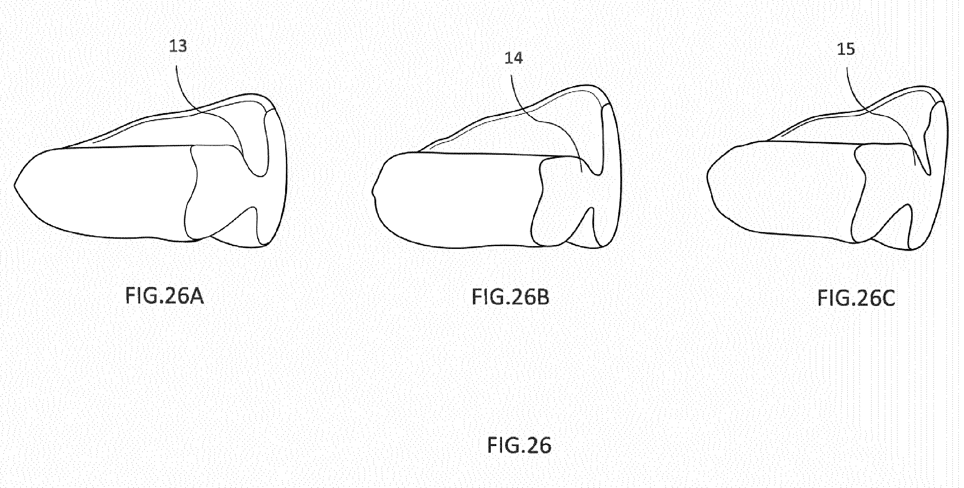

Advantageously according to the disclosure, as shown in Figure 26, the occlusal guides 13, 14, 15 of the devices 1, 10, 20 allow the correct positioning of the occlusal keys.

Moreover, advantageously according to the disclosure, the device 60 comprises an external grip element 61, for example a pacifier. This type of orthodontic elastic harmonizer device for dental-skull-facial apparatus 60 when the child insists on using a pacifier, even after three or four years of age (usually happens when the child has neuro-muscular tensions, also associated with nocturnal teeth grinding).

In addition, the device has a very good elasticity also as a de-tensional instrument, and for a very interceptive and preventive treatment especially on skull-facial skeletal structures. In fact, advantageously according to the invention, the material used for the construction of the devices 1, 10, 20, 20', 30, 40, 50 and 60 is elastic, and, in particular, is silicone or thermoplastic type, biocompatible and with a hardness of 53 or 58 or 62, measured in Shore scale.

In the case of the device 50 and 60, the hardness is equal to 45 measured in the Shore A scale.

Each type of material has its own specific function in relation to masticatory load and is, therefore, indicated for children and adults with a chewing force and different tensions.

According to an aspect of the disclosure, the materials of which the devices are made shown are selected from:
- The Dinabase, having three gradations of Shore consistency 53-58-62, quite effective and comfortable when worn and which presents no difficulty in processing;
- The Corflex, with three levels of Shore consistency 45-60-80. In particular, the material Corflex Soft 45 and 60 is indicated in tensional failure (muscle-tension) and neuro-muscular, while the Corflex 80 is more indicated in some forms of joint failure (ATM), and especially for purely orthopedic osteopathic action when lesions are present with the different deviations of the skull-facial structure.

These characteristics give the shown devices the ability to carry out a gingival and muscle massage action without a hazard of injury or excoriation of the mucous membranes during use.

The preferred embodiment of the disclosure according to the attached figures contemplates an embodiment of the device 1 suitable to a size of a standard arch. It is provided also the realization of the device 1 as described in different sizes to adapt to a small mouth, up to a mouth of larger dimensions.

From the operational point of view, the end user who uses the described devices, must place the device in own mouth in the correct position and can, for almost regular intervals and for a certain number of times a day, an operation of chewing the device. This chewing operation is realized in the action of the teeth of the upper and lower jaws (positioned in the concave space bounded by the vestibular flanges, palatal-lingual flanges and by the occlusal plane) that bite regularly the occlusal plane on both the upper and lower sides, generating multiple beneficial effects as will be described below. Depending on the case, the use of the device can also be associated to the use of the device itself with specific products in the form of gel to be affixed to the internal layer of the vestibular flanges, namely the parts that are in direct contact with the teeth and gums.

During the use, the device according to the disclosure procures an action on the upper maxilla, by positioning the jaw, through a specific stimulation relative to the type of conformation and geometry of the device itself, and provide muscular action well determined by the effect of elastic materials used. The orthodontic elastic harmonizer device for dental-skull-facial apparatus can have effect on the tension of the sutures and cranial structures, reconditioning and repositioning the location of the different cranial bones, and then of the whole the craniofacial system, with the consequent positioning of the teeth (relative to this new bone balance), by means of a real and proper gymnastics. Because, both the flanges and the occlusal planes extend up to cover all the teeth well attached to gingival ridges, forming a binary, both of the upper arch and of the lower part and determining a proprioceptive action of the gum areas, bone areas (periodontal) and on the teeth, the different dysmetria of the craniofacial structure are thus restored resulting in alignment of teeth badly positioned in the arch. In addition, the bimaxillary action of the orthopedic device is also decisive for the repositioning of the jaw, when a deviation of the same exists. The device is also arranged for the re-education of posture and correct the tongue swallowing, when there is an abnormal swallowing, through the inclined plane driving the tongue at the center of the lingual flange and the little button which act as a stimulus. In addition, the device acts simultaneously as expander and the dental arches, thanks to the activation of the elastic type due to the manufacturing material capable of procuring orthopedic action. Therefore, the orthodontic elastic harmonizer device for dental-skull-facial apparatus for its therapeutic validity, as well as to restore of skull-facial skeletal disorders, acts as a tool suited to the resolution of several other parafunctional, dental, occlusal, joint (TMJ), tensional neuro-muscular and postural pathologies, as well as in the rehabilitation of functions disturbed such as: swallowing, chewing, and speech, bruxism and night snoring. The operation of the device is done by motivating the patient to perform certain exercises, in order to obtain a true self-stimulation that will rebalance and harmonize the aforementioned disorders.

Therefore, the orthodontic elastic harmonizer device for dental-skull-facial apparatus according to the disclosure provides a more comprehensive and proper treatment on their class and any form of asymmetry, directing the treatment priority to get a good verticentrica and good harmonization of the dental arches acting in the three spatial planes (vertical, horizontal, and sagittal), taking into account the different type of shape of the arch and of the development of the stomatognathic apparatus in which the patient is at that precise moment and the most suitable as possible to the failure correction, performing the neural excitation (by means of the completely elastic effect of the orthodontic-elastic-harmonizer dental-skull-facial device) that is the basis of the clinical effects of the treatment, in order to eliminate all interferences and causes of a skeletal skull-facial altered and dysmetric system.

In addition, the orthodontic elastic harmonizer device for dental-skull-facial apparatus according to the disclosure can be used both by adults and by children.

Another advantage of the orthodontic elastic harmonizer device for dental-skull-facial apparatus according to the disclosure is to be an elastic functional device for the maxilla and the mandible effective in shaping the dental arches and do not determine possible trauma or damage to the teeth or the periodontium, not resulting invasive. The orthopedic rehabilitative action of the device is able to be reflected throughout the stomatognathic system (teeth, parodonte, masticatory muscles, joints (TMJ), cheeks, lips, tongue, innervation, vascularization and the maxillary and mandibular bones), and through its action mechanism, allows a true muscle gymnastic training in respect of the most suitable neuro-muscular function. The muscle relaxing action resulting in the elimination of tension and muscular contraction can, in fact, to get the re-harmonization of the jaws, teeth regain their place and the relationship between the bones of the skull improves.

Another advantage of the orthodontic elastic harmonizer device for dental-skull-facial apparatus according to the disclosure is to be non-invasive and easy to withstand even for the more sensitive patients.

In addition, the orthodontic elastic harmonizer device for dental-skull-facial apparatus according to the disclosure is indicated in patients with present ATM dysfunction (Temporo Mandibular joint), in extra-articular dysfunctions, temporomandibular meniscus and dental occlusion and associated to specific exercises, and it is able to correct the correct posture lingual. It is therefore possible to see an improvement of the various gold dental diseases, periodontal, skull-mandibular, neuro muscular and postural and restoration of the autonomic functions of the mouth, to correct swallowing, breathing, chewing, phonation.

In the end, the orthodontic elastic harmonizer device for dental-skull-facial apparatus according to the disclosure guides and stimulates the pulses generated by muscle movement causing the functional and physiological arousal developed, urging the formation of tissues, their adaptation and their morphology depending on the direction, intensity and frequency of these stimuli. At a functional level it is able to stimulate the growth of the condylar area, to correct the malocclusion and then reload neurologically and consequently regenerate the muscular system. With the vibrational effect obtained by means of specific exercises that represent a real gymnastics transmitted on all the craniofacial apparatus, it is crucial in producing a myo-relaxing action resulting in the elimination of the voltage and muscle contracture. It also produces a deep tissue massage that improves the removal of metabolic degradation of the inflammatory process and edematous and favors the normalization of the neuro-muscular system.

It is finally clear that the orthodontic elastic harmonizer device for dental-skull-facial apparatus here described and illustrated can be subject to modifications and variations without thereby departing from the protective scope of the present invention, as defined in the appended claims.

## Claims

1. Orthodontic elastic harmonizer device for dental-skull-facial apparatus (1, 10, 20, 20', 30, 40, 50, 60) comprising:
- at least a couple of vestibular flanges (2) extending up to cover maxilla and mandible teeth and gingival vestibular of the maxilla and mandible teeth;
- at least a couple of lingual-palatal flanges (6) extending to cover the palatal and lingual surfaces of the maxilla and mandible teeth and comprising a steep inclined plane (6a) extending from the back incisive plantar edge until lower frontal teeth configured to drive the tongue on the palate;
- at least an occlusal plane (3, 3', 3", 3''') to connect said flanges (2, 6); and
- concave and empty volumes (7) defined by said vestibular (2) and lingual-palatal flanges (6) and said occlusal plane (3, 3', 3", 3''');
**characterized in** comprising:
- protrusions (9) on each edge of a back portion of the top lingual-palatal flange (6) used as reference points configured to indicate the position of with respect to the arch of the patient, in correspondence of the molar position and a first and a second guide groove configured to accommodate removable ties therebetween on a lateral back portion of the upper surface of said occlusal plane (3, 3', 3", 3'''), the first guide groove placed at the corner between said occlusal plane (3, 3',3", 3''') and said vestibular flanges (2) and the second guide groove placed at the corner between the occlusal plane (3, 3',3", 3''') and the lingual-palatal flanges (6);
- an indentation (8) suitable to unload the back incisive papilla positioned in the top lingual-palatal flange (6); and
- an occlusal guide (13, 14, 15) configured as alignment means for aligning the upper teeth with the lower teeth.

2. Orthodontic elastic harmonizer device for dental-skull-facial apparatus (1, 10, 20, 20') according to claim 1, **characterized in that** the vestibular (2) and upper lingual-palatal flanges (6) are well approached between them by the occlusal plane (3) to form a teeth rail guide with a thickness at least of 2 mm, in the top frontal teeth area (canine incisors), of at least 5 mm in the back teeth area (premolars), and of at least 10 mm in the molar teeth most back area and that the vestibular (2) and bottom lingual-palatal flanges (6) are narrower than upper flanges.

3. Orthodontic elastic harmonizer device for dental-skull-facial apparatus (1, 10, 20, 20') according to claim 1, **characterized in that** the occlusal plane (3) have a front occlusal portion (3a, 3'a) more raised than a back occlusal portion (3b, 3'b).

4. Orthodontic elastic harmonizer device for dental-skull-facial apparatus (40) according to claim 1, **characterized in that** the occlusal plane (3") have a back occlusal portion (3"b) more raised than front occlusal portion (3"a) with a top maxilla slide forwards effect regard the mandibular occlusal.

5. Orthodontic elastic harmonizer device for dental-skull-facial apparatus (1, 10, 20, 20', 30, 40, 50, 60) according to claim 1, **characterized in that** the protrusion (9a, 19a, 29a, 39a, 49a, 59a, 69a) is a "tongue".

6. Orthodontic elastic harmonizer device for dental-skull-facial apparatus (60) according to claim 1, **characterized in** comprising an external gripping element (61) .

## Patentansprüche

1. Elastische kieferorthopädische Harmonisierungsvorrichtung für Zahn-Schädel-Gesichtsapparat (1, 10, 20, 20', 30, 40, 50, 60), umfassend:
- mindestens ein Paar vestibuläre Flansche (2), die sich nach oben erstrecken, um Ober- und Unterkieferzähne sowie gingival-vestibulär der Ober- und Unterkieferzähnen zu bedecken;
- mindestens ein Paar lingual-palatinaler Flansche (6), die sich so erstrecken, dass sie die palatinalen und lingualen Oberflächen der Ober- und Unterkieferzähne bedecken, und die eine steile geneigte Ebene (6a) umfassen, die sich von der hinteren Schneidezahnplantarkante bis zu den unteren Vorderzähnen erstreckt und so ausgelegt ist, dass sie die Zunge auf den Gaumen führt;
- mindestens eine Okklusionsebene (3, 3', 3", 3''') zur Verbindung der Flansche (2, 6) und
- konkave und leere Volumina (7), die durch die vestibulären (2) und lingual-palatinalen Flansche (6) und die Okklusionsebene (3, 3', 3'', 3''') definiert sind,
**dadurch gekennzeichnet, dass** sie Folgendes umfasst:
- Vorsprünge (9) an jeder Kante eines hinteren Abschnitts des oberen lingual-palatinalen Flanschs (6), die als Referenzpunkte verwendet werden und so ausgelegt sind, dass sie die Position in Bezug auf den Bogen des Patienten an der Molarenposition anzeigen, und eine erste und eine zweite Führungsnut, die so ausgelegt sind, dass sie entfernbare Bänder dazwischen an einem seitlichen hinteren Abschnitt der oberen Fläche der Okklusionsebene (3, 3', 3", 3''') aufnehmen, wobei die erste Führungsnut an der Ecke zwischen der Okklusionsebene (3, 3', 3", 3''') und den vestibulären Flanschen (2) und die zweite Führungsnut an der Ecke zwischen der Okklusionsebene (3, 3', 3", 3''') und den lingual-palatinalen Flanschen (6) angeordnet ist;
- eine Einbuchtung (8), die geeignet ist, um die im oberen lingual-palatinalen Flansch (6) positionierte hintere Schneidezahnpapille zu entlasten, und
- eine okklusale Führung (13, 14, 15), die als Ausrichtungsmittel für die Ausrichtung der oberen Zähne mit den unteren Zähnen ausgelegt ist.

2. Elastische kieferorthopädische Harmonisierungsvorrichtung für Zahn-Schädel-Gesichtsapparate (1, 10, 20, 20') nach Anspruch 1, **dadurch gekennzeichnet, dass** die vestibulären (2) und oberen lingual-palatinalen Flansche (6) dazwischen durch die Okklusionsebene (3) gut angenähert sind, um eine Zahnschienenführung mit einer Dicke von mindestens 2 mm im Bereich der oberen Frontzähne (Eckzähne), von mindestens 5 mm im Bereich der vorderen Backenzähne (Prämolaren) und von mindestens 10 mm im Bereich der hintersten Molaren zu bilden, und dass die vestibulären (2) und unteren lingual-palatinalen Flansche (6) schmäler sind als die oberen Flansche.

3. Elastische kieferorthopädische Harmonisierungsvorrichtung für Zahn-Schädel-Gesichtsapparate (1, 10, 20, 20') nach Anspruch 1, **dadurch gekennzeichnet, dass** die Okklusionsebene (3) einen vorderen Okklusionsabschnitt (3a, 3'a) aufweist, der stärker erhöht ist als ein hinterer Okklusionsabschnitt (3b, 3'b).

4. Elastische kieferorthopädische Harmonisierungsvorrichtung für Zahn-Schädel-Gesichtsapparate (40) nach Anspruch 1, **dadurch gekennzeichnet, dass** die Okklusionsebene (3'') einen hinteren Okklusionsabschnitt (3"b) aufweist, der stärker erhöht ist als der vordere Okklusionsabschnitt (3"a), mit einer Oberkiefer-Gleitwirkung nach vorn in Bezug auf die Unterkiefer-Okklusion.

5. Elastische kieferorthopädische Harmonisierungsvorrichtung für Zahn-Schädel-Gesichtsapparat (1, 10, 20, 20', 30, 40, 50, 60) nach Anspruch 1, **dadurch gekennzeichnet, dass** der Vorsprung (9a, 19a, 29a, 39a, 49a, 59a, 69a) eine "Zunge" ist.

6. Elastische kieferorthopädische Harmonisierungsvorrichtung für Zahn-Schädel-Gesichtsapparat (60) nach Anspruch 1, **dadurch gekennzeichnet, dass** sie ein äußeres Greifelement (61) umfasst.

## Revendications

1. Dispositif harmoniseur élastique orthodontique pour un appareil dentaire-crânien-facial (1, 10, 20, 20', 30, 40, 50, 60) comprenant :
au moins un couple de brides vestibulaires (2) s'étendant jusqu'à couvrir les dents maxillaires et mandibulaires et le vestibulaire gingival des dents maxillaires et mandibulaires ;
au moins un couple de brides linguales-palatales (6) s'étendant pour couvrir les surfaces palatales et linguales des dents maxillaires et mandibulaires et comprenant un plan incliné (6a) raide s'étendant du bord plantaire incisif arrière jusqu'aux dents frontales inférieures conçues pour entraîner la langue sur le palais ;
au moins un plan occlusal (3, 3', 3", 3''') pour raccorder lesdites brides (2, 6) ; et
des volumes (7) concaves et vides définis par lesdites brides vestibulaires (2) et linguales-palatales (6) et ledit plan occlusal (3, 3', 3", 3") ;
**caractérisé en ce qu'**il comprend :
des saillies (9) sur chaque bord d'une partie arrière de la bride linguale-palatale (6) supérieure servant de points de référence conçus pour indiquer la position par rapport à l'arcade du patient, en fonction de la position molaire et une première et une seconde rainure de guidage conçue pour recevoir des attaches amovibles entre elles sur une partie arrière latérale de la surface supérieure dudit plan occlusal (3, 3', 3", 3'''), la première rainure de guidage étant placée au coin entre ledit plan occlusal (3, 3', 3", 3''') et lesdites brides vestibulaires (2) et la seconde rainure de guidage étant placés au coin entre le plan occlusal (3, 3', 3", 3''') et les brides linguales-palatales (6) ;
une indentation (8) conçue pour décharger la papille incisive arrière positionnée dans la bride linguale-palatale (6) supérieur ; et
un guide occlusal (13, 14, 15) conçu sous la forme d'un moyen d'alignement pour aligner les dents supérieures avec les dents inférieures.

2. Dispositif harmoniseur élastique orthodontique pour un appareil dentaire-crânien-facial (1, 10, 20, 20') selon la revendication 1, **caractérisé en ce que** les brides vestibulaires (2) et linguales-palatales (6) supérieures sont bien rapprochées entre elles par le plan occlusal (3) pour former un rail de guidage de dents d'une épaisseur d'au moins 2 mm, dans la zone des dents frontales supérieures (incisives canines), d'au moins 5 mm dans la zone des dents postérieures (prémolaires) et d'au moins 10 mm dans la zone la plus en arrière des molaires et que les brides vestibulaires (2) et linguales-palatales (6) inférieures sont plus étroites que les brides supérieures.

3. Dispositif harmoniseur élastique orthodontique pour un appareil dentaire-crânien-facial (1, 10, 20, 20') selon la revendication 1, **caractérisé en ce que** le plan occlusal (3) comporte une partie occlusale (3a, 3'a) avant plus élevée qu'une partie occlusale (3b, 3'b) arrière.

4. Dispositif harmoniseur élastique orthodontique pour un appareil dentaire-crânien-facial (40) selon la revendication 1, **caractérisé en ce que** le plan occlusal (3") comporte une partie occlusale (3"b) arrière plus élevée que la partie occlusale (3"a) avant à un effet de glissement supérieur maxillaire vers l'avant par rapport à l'occlusal mandibulaire.

5. Dispositif harmoniseur élastique orthodontique pour un appareil dentaire-crânien-facial (1, 10, 20, 20', 30, 40, 50, 60) selon la revendication 1, **caractérisé en ce que** la saillie (9a, 19a, 29a, 39a, 49a, 59a, 69a) est une « languette ».

6. Dispositif harmoniseur élastique orthodontique pour un appareil dentaire-crânien-facial (60) selon la revendication 1, **caractérisé en ce qu'**il comprend un élément de préhension (61) externe.
